# EUROPEAN PATENT SPECIFICATION

(11) **EP 0 536 352 B1**
(45) Date of publication and mention of the grant of the patent: **17.12.2003**
(21) Application number: 92907420.1
(22) Date of filing: 02.04.1992
(51) Int. Cl.: C12N 15/49, A61K 39/21, G01N 33/569

(54) **THERAPEUTIC AND PREVENTIVE VACCINES FOR HIV**
THERAPEUTISCHER- UND PRÄVENTIVIMPFSTOFF GEGEN HIV
VACCINS THERAPEUTIQUES ET PREVENTIFS POUR LE VIH

(30) Priority: 02.04.1991 US 681624; 14.02.1992 US 837781
(43) Date of publication of application: 14.04.1993
(73) Proprietor: ALBERT EINSTEIN COLLEGE OF MEDICINE OF YESHIVA UNIVERSITY, Bronx New York 10461 (US)
(72) Inventor: RUBINSTEIN, Arye, Monsey, NY 10952 (US); BLOOM, Barry, R., Hastings-on-Hudson, NY 10706 (US); DEVASH, Yair, Princeton Junction, NJ 08550 (US); CRYZ, Stanley, CH-3176 Neuenegg (CH)
(74) Representative: VOSSIUS & PARTNER
(86) International application number: EP9200735
(87) International publication number: WO92017590

(56) References cited:
- EP-A- 0 402 088
- WO-A-90/03984
- WO-A-91/14449
- WO-A-99/17789
- LAROSA G.J. ET AL: 'Conserved sequence and structural elements in the HIV-1 principal neutralizing determinant' SCIENCE vol. 249, 24 August 1990, pages 932 - 935

## Description

### FIELD OF THE INVENTION

This invention relates to vaccines and immunotherapeutic treatments for use in inhibiting the transmission (transmission prevention) and treatment of HIV. Specifically, it relates to vaccines created by the conjugation of HIV-1 peptides and carriers. These peptides may be either single or repeated, linear or conformational amino acid sequences from the gp120 Principal Neutralizing Domain (PND) of HIV-1. Examples of amino acid sequences from the gp120 PND of HIV are KRIHIGPRAFYT, RHIHIGPGRAFYT, RRIHIGPGRAFYT, TRIHIGPGRAFYT, CTRPNYNKRKRIHIGPGRAFYTTKNIIGTIRQAHC, GPGRAFGPGRAFGPGRAFC, IYIGPGRAC, IAIGPGRAC, IHIGPGRAC, KRIHIGPGRAFYT, RSIHIGPGRAFYA, KSITKGPGRVIYA, KGIAIGPGRTLYA and SRVTLGPGRVWYT.

The most effective amino acid sequences from the gp120 PND of HIV are GPGRAFGPGRAFGPGRAFC, IYIGPGRAC, IAIGPGRAC. IHIGPGRAC, KRIHIGPGRAFYT, RSIHIGPGRAFYA, KSITKGPGRVIYA, KGIAIGPGRTLYA and SRVTLGPGRVWYT. The best carrier is Purified Protein Derivative (PPD) of tuberculin from Mycobacterium tuberculosis. Either a single peptide carrier conjugate alone or several different peptide carrier conjugates together in a "cocktail" may be used to vaccinate HIV-1 infected and uninfected individuals. After the vaccines are administered to individuals, antibody-containing fluid from the vaccinated individuals is used in an assay to detect whether high affinity/avidity neutralizing and/or protective HIV-specific antibodies are present in the fluid. The assay is an antigen-limited ELISA which contains a thimerosal-containing diluent and is capable of detecting high affinity/avidity neutralizing and/or protective HIV-specific antibodies. In addition, in vitro lymphocyte proliferative responses, IL-2 secretion upon incubation with PND and generation of cytotoxic lymphocytes (CTL) maybe used to determine whether the vaccines are effective against HIV. The conjugates which have induced production of the antibodies and/or induced PND lymphocyte proliferation, IL-2 or CTL are useful in the treatment and transmission prevention of HIV.

### BACKGROUND OF THE INVENTION

There have been recent advances in the use of retrovirus-derived vaccines for the treatment of HIV. Specifically, a formalin-inactivated whole HIV vaccine has been developed which has conferred protection in Macaques. Immunization with vaccines potentiated with albumin has resulted in the protection from clinical disease in eight out of nine monkeys challenged with infectious doses of HIV. Notably, protection could be achieved even in cases where entry of viruses is not prevented, suggesting that it may not be necessary to completely block infection in order to have a successful vaccine.

Whole killed HIV vaccines have also been beneficial in the treatment of chimpanzees who were previously infected by HIV. These chimpanzees appear to have cleared the HIV infection in their blood streams following the vaccinations. Post-exposure immunization in humans has also been studied. These tests suggest that immunization may be used to protect humans from HIV infections, and also to treat humans who have already been infected with the virus. However, whole virus vaccines may contain infectious particles. As a result, it may be safer to use essential components of the virus to confer protection. Epitopes of the virus are one example of a safer, essential component of the virus. More recent studies have confirmed that partial protection from infection can be achieved also by gp120 and gp160 derived vaccines. (Desrosiers, R.C. et al. Proc. Nat'l. Acad. Sci. USA, 86:6353 (1989), Kestler, et al. Science, 248:1109 (1990); Murphey-Corb, M. Science, 246:1293 (1989)).

It has long been recognized that peptide epitopes of amino acids conjugated to immunogenic carriers can elicit high levels of high affinity antipeptide antibodies. (See Talwar, G.P., Bloom, B. et al., "Biological and Clinical Aspects of Reproduction", Exceotor Med. Series 394:2224-2232 (1987), in which the beta chain of human chorionic gonadotropin was conjugated to tetanus toxoid to produce an antifertility vaccine.) The carriers to which the peptides are conjugated in this invention have all been used as immunogenic carriers in animals, and some have been used in humans.

The purified protein derivative (PPD) of tuberculin from Mycobacterium tuberculosis is a unique immunologic reagent, because virtually everyone in the world with a functional immune response who has been exposed to BCG or M. tuberculosis infections will have a T-cell mediated, delayed-type hypersensitivity response to minute amounts of PPD. Tuberculin-PPD conjugates have been utilized in the past. Mice pre-sensitized or "primed" with BCG can produce high levels of antibodies to peptide or carbohydrate epitopes conjugated to PPD. Of particular interest are studies on the NANP repeating epitope of the P. falciparum circumsporozoite antigen, which is immunogenic in only two strains of mice. Conjugating the NANP repeating peptide to PPD elicits the production of antibody titers greater than 1:1000 in genetically non-responder strains to the NANP epitope. This degree of response is comparable to that seen in responder strains given the peptide conjugate in complete Freund's adjuvant. (See Lussow et al., "Use of Tuberculin Purified Protein Derivative-Asn-Ala-Asn-Pro Conjugate in Bacillus Calmette-Guerin Primed Mice Overcomes H-2 Restriction of the Antibody Response and Avoids the Need for Adjuvants," Proc. Nat'l Acad. Sci. USA 87 (1990)).

Pseudomonas aeruginosa exotoxin A (toxin A) has been used effectively as a carrier in conjugate vaccines. Conjugates made with this carrier have higher immunogenicity, especially when coupled with the recombinant protein R32 to create an immune response against the sporozoite stage of Plasmodium falciparum. Pseudomonas aeruginosa exotoxin A may be purified from the supernatant of fermentor-grown cultures of Pseudomonas aeruqinosa PA 103.

Keyhole Limpet Hemocyanin (KLH) is a high molecular weight protein which is purified from megathura crenulata. KLH has many available primary amines from lysine residues which facilitate protein conjugation. KLH is highly immunogenic, and because of its availability of primary amines, is ideal for protein conjugation.

Tetanus and diptheria toxoids have also been used successfully as protein carriers. Diptheria toxoid has been used with a synthetic 31 amino acid N-terminal peptide. Both tetanus toxoid and diptheria toxoid have proved to be effective carriers in humans for the poorly immunogenic carbohydrate antigen of Hemophilus influenza b.

The recombinant core antigen of hepatitis B has the capability of self-assembling into 27 millimeter particles which are highly immunogenic in experimental animals. These HBV core particles may be conjugated directly with peptides, using recombinant DNA technology. Fusion proteins can be produced between the HBV core antigen and defined sequence peptides with high epitope density, which lead to high titer antibodies, as well as to long lasting neutralizing antiviral immunity. Hepatitis B core antigens and self-assembled HBc-HIV peptide fusion protein may be used as protein carriers.

It has been established that the major antigenic component of the mycobacterial cell wall is a protein which consists of a polypeptide monomer of between 10 and 16 Kd, the amino terminal sequence of which reveals that it is related to the GroES heat-shock protein present in many bacteria. It has been indicated that the major antigenic component of mycobacteria recognized by CD4+ T-cells is associated with the cell wall. BCG cell wall (purified) may be used as a protein carrier. BCG may also be used to prime animals or humans prior to vaccination. BCG priming enhances the humoral and cellular responses induced by vaccination.

Currently, the only adjuvant licensed for use in man is alumina. In the present invention, alumina may be used with the conjugates which include the carriers Pseudomonas aeruginosa exotoxin A, KLH, and tetanus and diptheria toxoids. Aluminum-based gels such as Al(OH)₃ and AlPO₄, as well as liposomes may be used as adjuvants with the carrier Pseudomonas aeruginosa exotoxin A in the present invention. PPD conjugates can be given in saline with no further adjuvants to tuberculin-positive individuals. For the BCG cell wall and HBc antigen conjugates, it is likely that adjuvants would be required. Ribi adjuvant containing trehalose dimycolate and 2% squalene may be used as an adjuvant. Alternatively, if studies on the long-term safety of incomplete Freund's adjuvant or ISCOM's adjuvant indicate their safety and efficacy in humans, these adjuvants may be used. Microencapsulation technology using polyactide/polyglycolide biodegradable polymers may also be used as an adjuvant.

None of the methods developed to date, including the use of carriers, have been successful in the treatment and transmission prevention of HIV. It is an object of the present invention to provide peptide carrier conjugate vaccines to treat and inhibit transmission of HIV, and to provide methods for the production of such vaccines.

### BRIEF DESCRIPTION OF THE DRAWINGS

Figure 1 represents the immunoreactivity of mice vaccinated with conjugates of KLH and five different peptides: 282 (GPGRAFGPGRAFGPGRAFC), 283 (IYIGPGRAC), 284 (IAIGPGRAC), 285 (IHIGPGRAC) and MN (KRIHIGPGRAFYT).
Figure 2 represents the affinity/avidity of mice with the highest titers of antibody to conjugates of KLH and peptides 282 (GPGRAFGPGRAFGPGRAFC), 283 (IYIGPGRAC), 284 (IAIGPGRAC) and 285 (IHIGPGRAC).

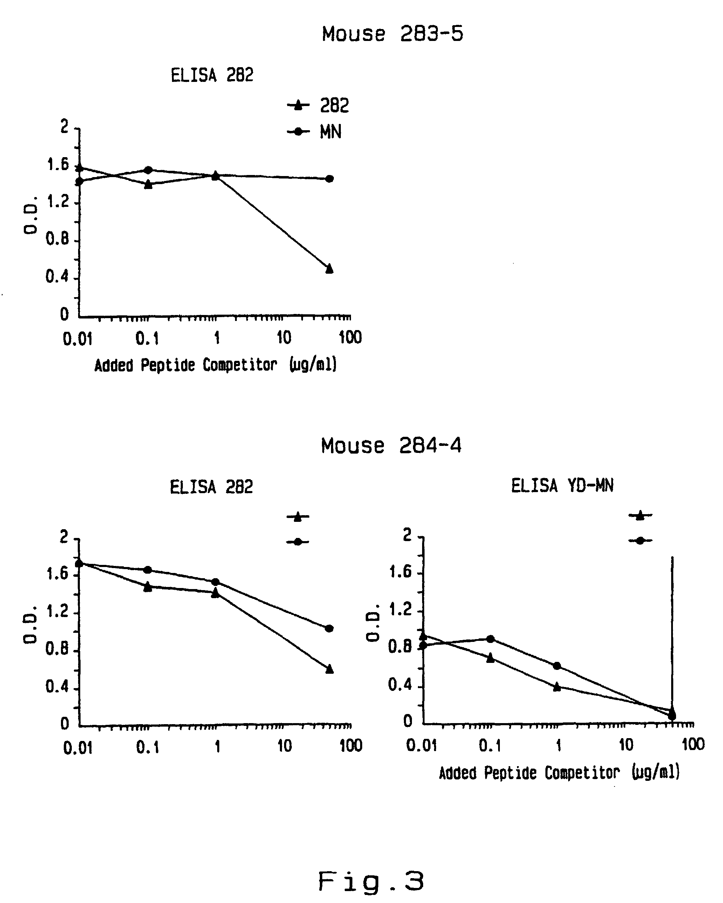

Figure 3 represents the results of competition between peptides 282 (GPGRAFGPGRAFGPGRAFC) and MN (KRIHIGPGRAFYT) with peptide 283 (IYIGPGRAC) in mouse 283-5. Peptide 282 competed effectively, and peptide MN did not. Figure 3 also represents the results of competition between peptides 282 and MN with peptide 284 (IAIGPGRAC) in mouse 284-4. Both peptides 282 and MN competed effectively.

### SUMMARY OF THE INVENTION

This invention relates to conjugates made up of (1) peptides with amino acid sequences similar to the gp120 Principal Neutralizing Domain (PND) of HIV and (2) carriers which enhance immunogenicity, such conjugates to be used in vaccines for the treatment and transmission prevention of HIV. In one embodiment of this invention, the peptides are KRIHIGPRAFYT, RHIHIGPGRAFYT, RRIHIGPGRAFYT, TRIHIGPGRAFYT, CTRPNYNKRKRIHIGPGRAFYTTKNIIGTIRQAHC, GPGRAFGPGRAFGPGRAFC, IYIGPGRAC, IAIGPGRAC, IHIGPGRAC, KRIHIGPGRAFYT, RSIHIGPGRAFYA, KSITKGPGRVIYA, KGIAIGPGRTLYA and SRVTLGPGRVWYT.

In a preferred embodiment of this invention, the peptides are GPGRAFGPGRAFGPGRAFC, IYIGPGRAC, IAIGPGRAC, IHIGPGRAC, KRIHIGPGRAFYT, RSIHIGPGRAFYA, KSITKGPGRVIYA, KGIAIGPGRTLYA and SRVTLGPGRVWYT.
In another preferred embodiment, the carrier is PPD. Individuals to be vaccinated who are PPD-negative are primed with BCG four to six weeks prior to vaccination with the conjugates in order to enhance the immune reaction induced by vaccination with the peptide carrier conjugates. If an individual is PPD-positive, it is not necessary to prime that individual with BCG.

The vaccine may comprise either a single peptide carrier conjugate alone, or a cocktail of several different peptide carrier conjugates. The vaccine may be administered in liquid form once or several times, or may be administered in a timed-release or pulse-release mechanism. After vaccination with the conjugate, antibody-containing fluid is extracted from the vaccinated individual and assayed in an antigen-limited ELISA which contains a thimerosal-containing diluent and selects for high affinity/avidity neutralizing and/or protective HIV-specific antibodies. Further, in vitro lymphocyte proliferative responses, IL-2 secretion upon incubation with PND and generation of cytotoxic lymphocytes (CTL) may be used to determine whether the vaccines are effective against HIV. The conjugates which have induced the production of such antibodies and/or induced PND lymphocyte proliferation, IL-2 or CTL are useful in the treatment and transmission prevention of HIV.

### DETAILED DESCRIPTION OF THE INVENTION

This invention is directed to peptide carrier conjugate vaccines to be used in the treatment and transmission prevention of HIV. It comprises the conjugation of peptides with amino acid sequences similar to those of the gp120 PND of HIV and carriers to form vaccines, priming animals with BCG, immunizing animals with the vaccines, extracting antibody-containing fluid from the immunized animals and assaying the antibody-containing fluid in an antigen-limited ELISA which contains a thimerosal-containing diluent and selects for high affinity/avidity neutralizing and/or protective HIV-specific antibodies. The vaccines which have induced the production of the high affinity/avidity neutralizing and/or protective HIV-specific antibodies and/or induced proliferative responses and/or IL-2 secretion upon in vitro exposure to PND are useful in the treatment and transmission prevention of HIV.

Carriers which may be used for conjugation with the peptides of this invention include purified protein derivative (PPD) of tuberculin from Mycobacterium tuberculosis, Pseudomonas aeructinosa exotoxin A, keyhole limpet hemocyanin (KLH), tetanus toxoid, diptheria toxoid, hepatitis B core antigen and Bacillus Calmette-Guerin (BCG) cell wall. The preferred carrier of this invention is PPD. In addition, an adjuvant, such as alumina, Ribi, Freund's, Iscom's or microencapsulation technology adjuvant may be used with all of these carriers.

In order to prepare the peptides of this invention, it is necessary to determine which strains of HIV infect individuals and which peptides of each strain would most effectively induce the production of high affinity/avidity neutralizing and/or protective HIV-specific antibodies. There are many different strains of HIV, and different strains are prevalent in different geographic areas. Therefore, the peptides in the vaccines of this invention may be geographically specific. For example, the MN strain of HIV has a high degree of prevalence in the United States. Therefore, peptides from the MN strain of HIV are effective in conjugates used to vaccinate individuals in the United States. These particular peptides and conjugates may not be effective in different geographic areas where other HIV strains are prevalent, and may have to be adjusted according to the most prevalent strains evolving in certain geographic areas. Another example is that high affinity/avidity neutralizing and/or protective HIV-specific antibodies against the gp120 PND of the MN strain of HIV have been associated with protection from materno-fetal HIV transmission in the United States. Therefore, peptides from the MN strain of HIV may be used in conjugates to prevent materno-fetal transmission of HIV in the United States.

The peptides of this invention are from the gp120 PND of HIV. The peptides selected may include amino acids 116-131, 307-319 and 470-490 of gp120 of MN-HIV. The most effective sequences to induce the production of high affinity/avidity neutralizing and/or protective HIV-specific antibodies, which are the preferred peptide sequences for the conjugates of this invention, are as follows:

Because soluble peptides are poor immuaogens due to their lack of T and B-cell reactive epitopes and their low molecular weight, the peptides of this invention are conjugated with different carriers to enhance immunogenicity. The methods by which these peptides are conjugated with the carriers include disulfide linkages through a C terminal peptide cysteine linkage, coupling with glutaraldehyde solution for two hours, coupling with tyrosine, or coupling with water soluble carbodiimide.

Where the carrier is PPD, PPD-negative individuals should be primed with BCG prior to conjugate vaccination. In order to determine whether an individual is PPD-negative or PPD-positive, either proliferative response testing or skin testing may be performed. For proliferative response testing, if an individual exhibits proliferative responses in vitro to peripheral blood lymphocytes to PPD, that individual is PPD-responsive. For skin testing, an individual should be exposed to intradermal 5 TU and if negative to 20 TU of PPD. If an individual is PPD-positive, there is no need to prime with BCG. If an individual is considered PPD-negative, that individual should receive a BCG immunization four to six weeks prior to conjugate vaccination. After BCG immunization, PPD testing should again be performed. If the results of the PPD test are positive, then the conjugate vaccine is administered to the individual.

If individuals are HIV-negative and PPD-negative, there are no limitations on BCG priming. However, if individuals are HIV-positive and PPD-negative, they may be primed with BCG only if they are still asymptomic and/or their CD4 T-cell counts exceed 200. Individuals who are HIV-positive, PPD-negative and exhibit advanced symptoms should not be primed with BCG. In addition, it is recommended that the standard British or Japanese BCG vaccines be used for priming, since these vaccines rarely induce adverse reactions in individuals.

The peptide-carrier conjugates of this invention may be administered in vaccine form, suppository form, intradermally, subcutaneously, orally, or by any other suitable route. The vaccines may comprise either a single peptide carrier conjugate or a cocktail of several different peptide carrier conjugates, and may be administered in liquid form or in timed-release, pulse-release or slow-release mechanisms, such as virosomes. virosomes encase viral-specific antigens in their systems and then react strongly with macrophages. Such virosomes may comprise 1-10 µg of PND peptide coupled to PPD or influenza hemagglutinin (HA), or PND in a free state associated with but not covalently coupled with PPD or HA, 1-10 µg of HA isolated from a human strain of influenza virus, 0.1-1 µg neuraminidase (NA) isolated from a human strain of influenza virus, 0.25-0.75 mg kephalin and 100-140 mg lecithin.

Either a single vaccination or multiple vaccinations with the peptide carrier conjugates may be used to induce the production of high affinity/avidity neutralizing and/or protective HIV-specific antibodies. The preferred dose range is 50-500 µg of peptide per conjugate. At a later date, antibody-containing fluid is extracted from the vaccinated individuals. The antibody-containing fluid is then assessed in an assay. The assay contains a thimerosal-containing diluent and is an antigen-limited ELISA which selects for high affinity/avidity neutralizing and/or protective HIV-specific antibodies.

In order to produce this assay, microplate wells are covered with the antigen which the antibodies are reactive to, such as MN-PND, or another PND antigen derived from other seroprevalent HIV strains, in a decreasing coating concentration series as follows:

| | |
|---|---|
| Row A - | 500 ng/ml |
| Row B - | 100 ng/ml |
| Row C - | 50 ng/ml |
| Row D - | 10 ng/ml |
| Row E - | 5 ng/ml |
| Row F - | 1 ng/ml |
| Row G - | 0.5 ng/ml |
| Row H - | 0 ng/ml |

Where the vaccine comprises a cocktail of different peptides conjugated to carriers, all of the wells in the plate are covered with all of the peptides in the vaccine in order to determine whether there was induction of high affinity/avidity neutralizing and/or protective HIV-specific antibody production. If such antibodies were produced, a second ELISA is performed, wherein each row of the plate is covered with a separate peptide from the cocktail.

Next, antibody-containing fluid is removed from the vaccinated individuals, diluted with antibody-containing fluid diluent to a ratio of 1:21 (sample: diluent) and added to the series of wells. The antibody-containing fluid diluent comprises a formulation of additives in a buffered solution containing thimerosal. An example of such antibody-containing fluid diluent is 0.1-0.5% caesin in PBS containing 0.05% Tween-20 (pH 7.4), 0.001% rhodamine and thimerosal. Any antibody-containing fluid may be used. Examples of antibody-containing fluid are serum, plasma, cerebral fluids and mucosal fluids. A negative control, such as anti-HIV negative human serum, and a positive control, such as anti-HIV positive human serum or anti HIV-MN-PND monoclonal antibody should also be added to wells in the ELISA plate. The plate should then be covered and incubated for 60 minutes at 37°C.

After incubation, the plate should be washed 5-6 times using diluted plate wash solution (phosphate buffered saline, pH 7.4 with 0.05% Tween-20 diluted with deionized water containing chloracetemide at 1:10). Use of an automatic plate washer is recommended. After washing, 100 µl per well of anti-human conjugate such as peroxidase-conjugated (goat) Fabl anti-human IgG, anti-human IgA or anti-human secretory component (SC), diluted to 1:100,000 in conjugate diluent is added to the wells. Any conjugate diluent may be used. The conjugate diluent may comprise, for example, a formulation of additives in a buffered solution which is typically added to antibody conjugate solutions by those skilled in the art. Subsequently, the wells are incubated for 60 minutes at 37°C and washed 5-6 times using the diluted plate wash solution. (Again, use of an automatic plate washer is recommended.)

After washing, 200 µl of substrate such as O-phenylenediamine (o-PD) tablets, diluted (to 1 tablet per 12 ml of diluent) with hydrogen peroxide in a citrate buffer is added per well. The wells are then incubated in the dark for 30 minutes at room temperature. The reaction is then stopped by adding 50 µl per well of 4N sulfuric acid. The plate may then be read in a microplate spectrophotometer at an absorbance of 492nm. (Use of a 620 nm reference filter is recommended.)

For the controls, the human negative control values should average around 0.050. The cutoff should be around 0.100. The human positive control should filter out at least to the fifth well (5 ng/ml).

For the antibody-containing fluid, if the absorbance value is greater than the cutoff in the fourth row or less, the antibody in the fluid is low affinity. If the absorbance value is greater than the cutoff in the fifth row or more (5 ng/ml), the antibody is medium affinity. If the absorbance value is greater than the cutoff in the fifth row or more (less than or equal to 5 ng/ml), the antibody is high affinity. A displacement assay may then be set up to ascertain the high affinity antibodies.

The affinity of the elicited antibodies (in vivo and in vitro) may be further assessed using the PHARMACIA BIACORI System, which is a biosensor-based technology (Biospecific Interaction Analysis, (BIA) which assesses biomolecular interactions to the picomolar range without the need for labels (radioactive or fluorescent). BIA measures the KA and KD of molecules bound to a surface with molecules in free solutions. By reflecting a beam of light on a miniature sensing surface, it is possible to obtain quantitative kinetic data about antigen/antibody binding (association-dissociation constraints).

The conjugate vaccines which have induced the production of high affinity/avidity neutralizing and/or protective HIV-specific antibodies, such antibodies being selected for by the ELISA, may then be used for the treatment and transmission prevention of HIV.

### EXAMPLE I-Immunization of Mice with Conjugates of Peptides Coupled to Carriers KLH, PPD, Avidin and Tetanus.

Mice were immunized with peptide KRIHIGPGRAFYT (MN peptide or MN-PND) coupled to different carriers. Five mice were immunized with the MN peptide coupled to Keyhole Limpet Hemocyanin (KLH) as described in Methods Enzymol., 70:159 (1980). The mice were bled and antibody titers directed against MN-PND were determined by an ELISA specific for MN-PND. Serum at a 1:1400 dilution obtained from mice immunized with the KLH-MN-PND conjugate had optical densities of 3.85 (K0), .598 (K1), 1.762 (K2), .282 (K3), and .356 (K4) with a background of less than 0.1. Four months later, the mice were boosted with KLH-MN-PND and then the sera was assayed for reactivity. This data is outlined in Table 1. All of the mice immunized had high titers and high affinity antibodies to MN-PND. Mouse K4 was sacrificed. Its spleen was harvested and used to develop monoclonal antibodies to MN-PND. Monoclonal antibodies recognizing MN-PND were generated, some of which were neutralizing. Other mice were immunized with PPD-MN-PND, avidin-MN-PND and tetanus-MN-PND conjugates. Serum from these mice had no significant activity directed against MN-PND.

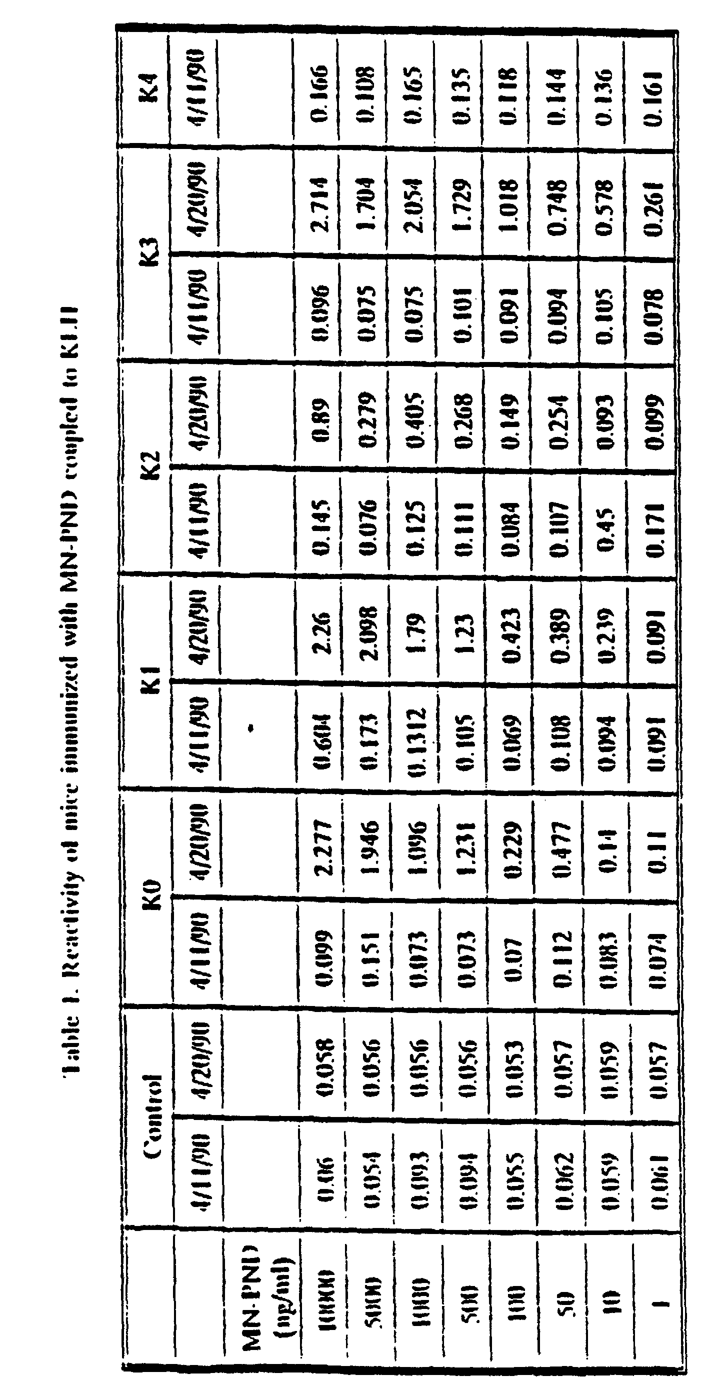

### EXAMPLE II-Immunization of Mice with Conjugates of Peptides Coupled to Carrier Toxin A.

### Production of PND-Toxin A Conjugate

One gram of solid carbodiimide was added to 100 mg of toxin A derivitized with adipic acid dehydrazide (TA-ADH, 5.9 mg/ml in phosphate buffered saline, pH 7.4; PBS). The pH was adjusted to 4.8 by the addition of 0.3N HCl. To this mixture 50 mg of MN-PND peptide in 2.5 ml of pyrogen-free water was slowly added under constant stirring. The mixture was stirred for 3 hours at ambient temperature. The mixture was filter sterilized using a 0.45 µm filter unit and passed through a Sephadex G75 column to separate the conjugate from reactants. The conjugate-containing fraction which eluted in the void volume was concentrated, dialyzed against PBS and filter sterilized. The conjugate was diluted to 200 µg total protein/ml in PBS and absorbed into Al(OH)₃.

### Immunization Studies

A group of 3 guinea pigs was vaccinated intramusculary on days 0 and 14 with 50 µg of absorbed conjugate. Serum samples were obtained on days 0, 14 and 28. An anti-MN-PND peptide ELISA was performed by coating plates with MN-PND peptide, reacting wells with serial dilutions of antisera, and after washing, reacting plates with an anti-guinea pig IgG antibody. As shown in Table 2 below, this conjugate did not stimulate an anti-MN-PND antibody response.

**TABLE 2**

| Day | mean ELISA titer |
|---|---|
| 0 | 1,4 |
| 14 | 1,8 |
| 28 | 1,0 |

### EXAMPLE III-Immunization of Mice, Rabbits and Guinea Pigs with Conjugates of Peptides Coupled to Carrier Toxin A.

Mice, rabbits and guinea pigs were immunized with MN-PND coupled to Pseudomonas aeruginosa exotoxin A (toxin A) with a ratio of peptide to carrier of 3:1. In addition, the MN-PND lacked a tyrosine residue next to the carboxy terminus. The animals were immunized on days 0 and 14. Sera was obtained at days 0, 14, and 28. The doses used were 5 and 25 µg in the guinea pigs, 5, 10 and 50 µg in the rabbits and 2 and 10 µg in the mice. The immunizations were given as either conjugate alone or in alum. None of the sera obtained from these immunizations had any reactivity to MN-PND. This was probably due to either the immunogenicity of toxin A, coupling problems or the dosage used.

### EXAMPLE IV-Immunization of Guinea Pigs with Conjugates of Peptides Coupled to Carrier PPD.

### Preparation of PPD-MN-PND Conjugate

4 mg of MN-PND in 400 µl of sterile distilled H₂0 was mixed with 4 ml of sterile PPD-RT23 (1 mg/ml). Carrier PPD-RT23 was obtained from Statens Serum Institute, Copenhagen, Denmark. Carrier PPD-298 was obtained from Connaught Laboratories, willowdale, Toronto. PPD-298-H2O signifies that the conjugation of the PND-MN peptide and the PPD-298 carrier was performed in water. PPD-298-PBS signifies that the conjugation of the PND-MN peptide and the PPD-298 carrier was performed in PBS. 45 µl of 0.2% glutaraldehyde was then added to the PPD-MN-PND solution, mixed by vortexing and incubated in the dark at 22°C for 30 min. An additional 20 µl of 0.2% glutaraldehyde was added and the solution was incubated for 90 min. The solution appeared opalescent at this time. The reaction mixture was transferred asceptically into a sterile dialysis tubing and dialysed against 1 liter of sterile PBS at 4°C for 24 hours. The concentration of PPD was calculated by dividing the total amount of PPD added to the reaction mixture by the final volume of the sample after dialysis. An aliquout of the conjugate was diluted in sterile pyrogen-free PBS to 20 µg of PPD per ml (∼1000E/ml) for determining the sterility and general safety of the vaccine preparation according to the guidelines of European Pharmacopea. The remainder of the sample was kept in undiluted form at 4°C.

### Immunogenicity of PPD-MN-PND Conjugate in Guinea Pigs

Groups of 5 guinea pigs were primed with 10⁶ CFU of BCG or saline. The animals were then immunized with 10 or 50 µg of PPD-MN-PND conjugate in 0.1 ml PBS 14 and 28 days after priming. The animals were bled at 14, 28 and 42 days after priming. The sera was then assayed in a MN-PND ELISA to determine immunogenicity of the PPD-MN-PND conjugate.

### Protocol for MN-PND HIV ELISA

Dynatech polystytene plates were coated with 100 µl of a 1.0 µg/ml of MN-PND (in carbonate buffer, pH 9.6) at 22°C overnight. The solution was removed and then blocked with 100 µl of a 1 mg/ml solution of Bovine Serum Albumin (BSA) in PBS (pH 7.4) for 1 hour at 22°C. A 100 µl of 2-fold serial dilutions of sera in PBS-tween (ph 7.4) starting at 1:20 were dispensed into each well and the plates incubated for 3 hours. The plates were washed 3 times with 300 µl of PBS-tween. 100 µl of peroxidase-conjugaied rabbit anti-guinea pig IgG (1:2500 in PBS tween, Nordic Immunology, Tilburg, The Netherlands) was added to each well and the plates were incubated for 2 hours. The plates were washed three times in PBS-tween and 100 µl of p-nitrophenyl phosphate (Merck) was added to the wells. The plates were then incubated for 60 min. at 22°C. The absorbances at 405 nm was measured using a Dynatech MR5000 Microtiter Plate Reader (Dynatech, Switzerland). The serum dilution at the linear part of the titration curve (between 0.15 and 0.6) was multiplied by the reciprocal corresponding dilution of the serum and expressed as ELISA units.

As shown in Table 3 below, priming of the guinea pigs with BCG and subsequent immunization with the PPD-MN-PND conjugate induced the production of high affinity/avidity neutralizing and/or protective HIV-specific antibodies. The antibody titers of the guinea pigs primed with BCG were much higher than the titers of the guinea pigs not primed with BCG, indicating that priming with BCG prior to conjugate immunization causes the immune system to respond better to conjugate immunization, thereby more effectively inducing the production of high affinity/avidity neutralizing and/or protective HIV-specific antibodies. The antibody titers of the guinea pigs not primed with BCG (group d) were not statistically higher than guinea pigs not immunized with the PPD-MN-PND conjugate vaccine (group a).

**TABLE 3**

| Immunogenicity of PND-MN-PPD Conjugates in BCG Primed and Unprimed Guinea Pigs | | | |
|---|---|---|---|
| Vaccine | Dose (µg) | Geometric Mean Elisa unit (range) | |
| -- | -- | 1.6 | (1.2-2.7)^{a} |
| PPD-MN-PND | 10 | 2.4 | (2.14-2.78)^{d} |
| PPD-MN-PND | 50 | 6.0 | (5.68-6.12)^{d} |
| PPD-MN-PND | 10 | 121.1 | (5.40-462.1)^{b} |
| PPD-MN-PND | 50 | 14.1 | (5.56-25.68)^{c} |
| RT23 | 10 | 21.0 | (6.7-70.8)^{b} |
| RT23 | 50 | 180.5 | (49.4-692.5)^{b} |
| PPD-298-H2O | 10 | 31.1 | (4.9-181.9)^{b} |
| PPD-298-H2O | 50 | 142.2 | (31.0-393.0)^{c} |
| PPD-298-PBS | 10 | 22.8 | (6.3-232.0)^{c} |
| PPD-298-PBS | 50 | 63.2 | (9.6-858.9)^{c} |

| | | | |
|---|---|---|---|
| a. Geometric Mean Elisa units of Pre immune sera from 4 guinea pigs. | | | |
| b. Geometric Mean Elisa units of post immune sera from 4 guinea pigs. | | | |
| c. Geometric Mean Elisa units of post immune sera from 3 guinea pigs. | | | |
| d. Guinea pigs not primed with BCG. | | | |

### EXAMPLE V-Immunization of Guinea Pigs and Mice with Conjugates of Peptides Coupled to Carrier PPD.

Guinea pigs and mice were immunized with MN-PND coupled to 2 types of PPD. Some of the PPD-immunized animals were first primed with BCG. Table 4 shows the immunoreactivity of the animals to MN-PND. Table 5 shows the titration of serum reactivity to MN-PND . Table 6 shows the affinity to MN-PND as measured by antigen-limited ELISA. Sample #6474 and Sample #6469 were assayed for neutralization activity. Results obtained indicated that neither of the sera had neutralizing activity to MN-HIV.

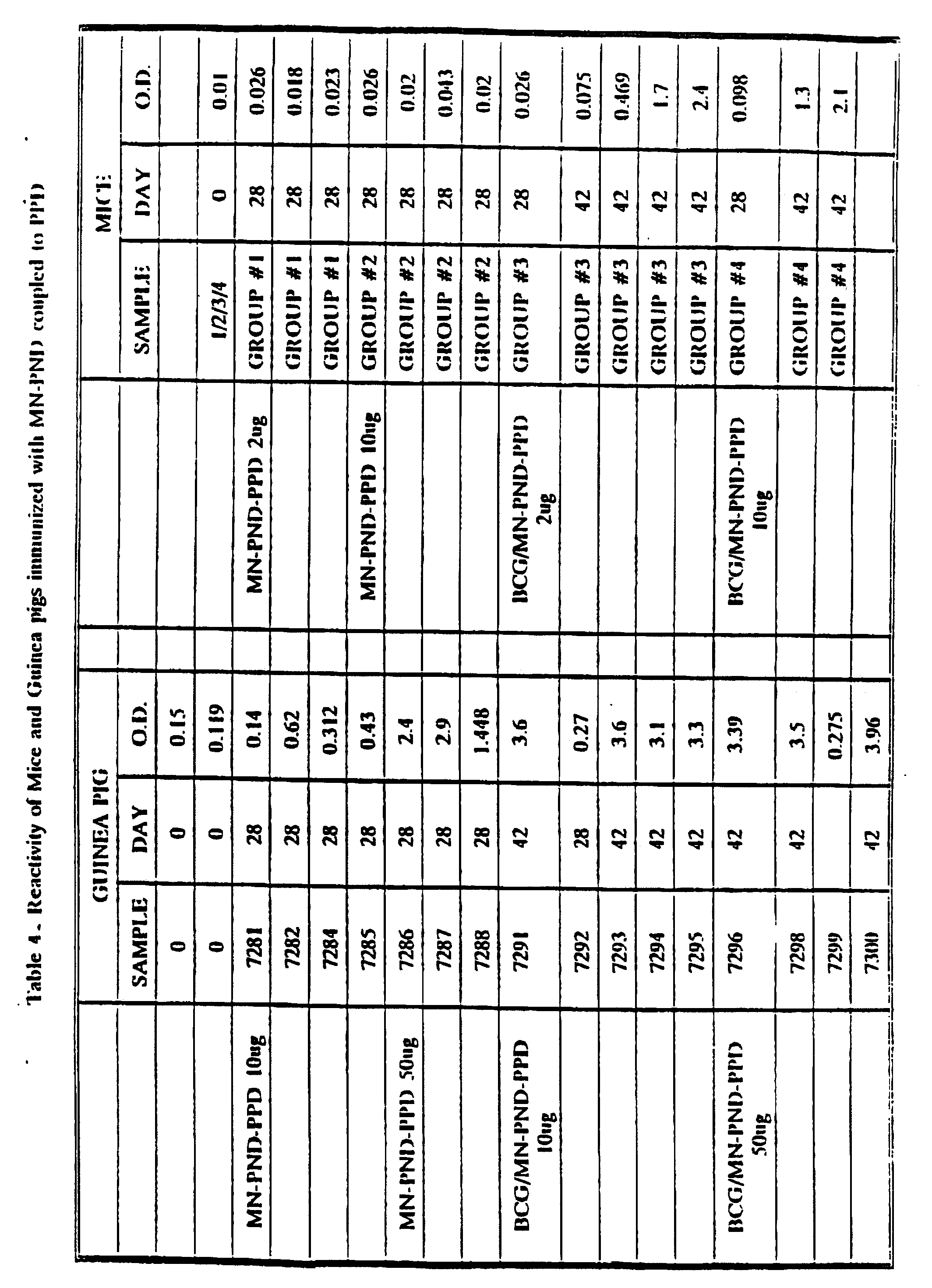

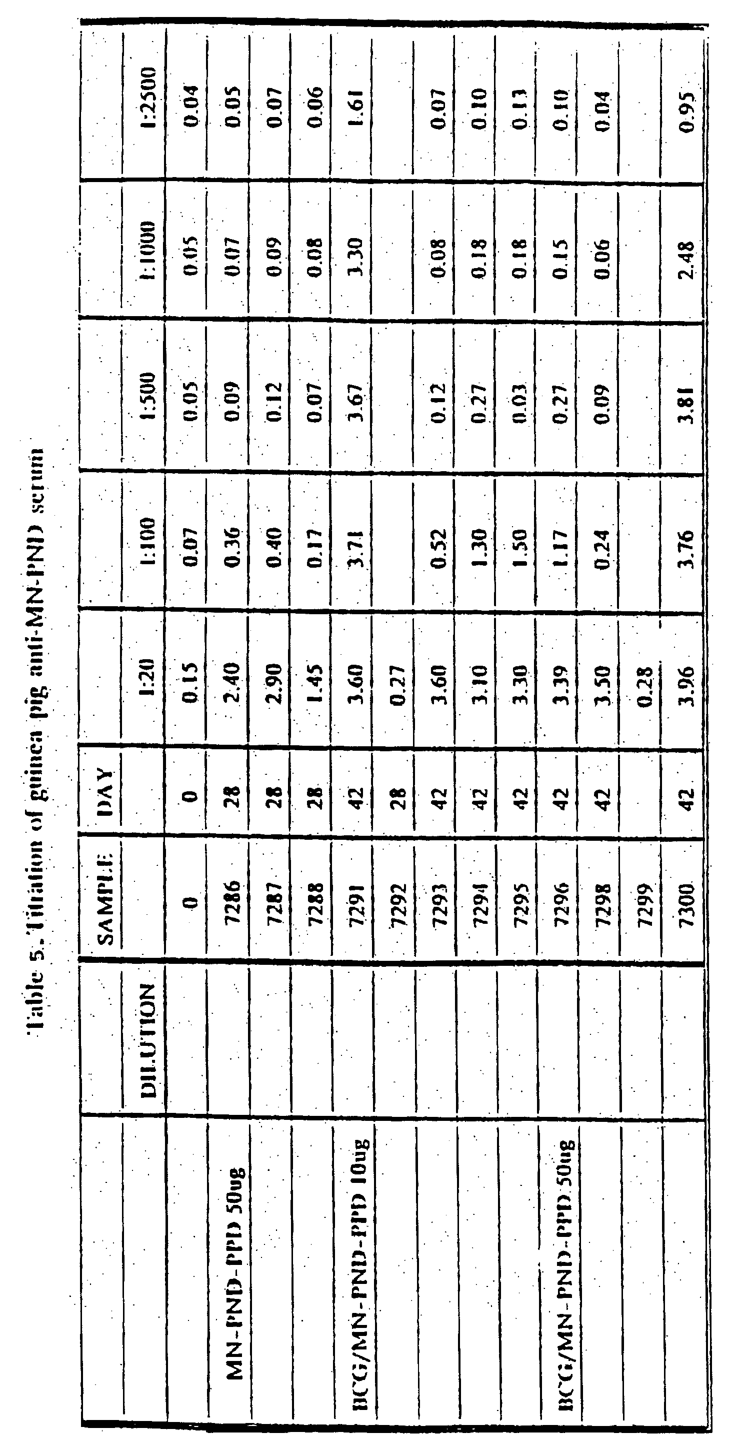

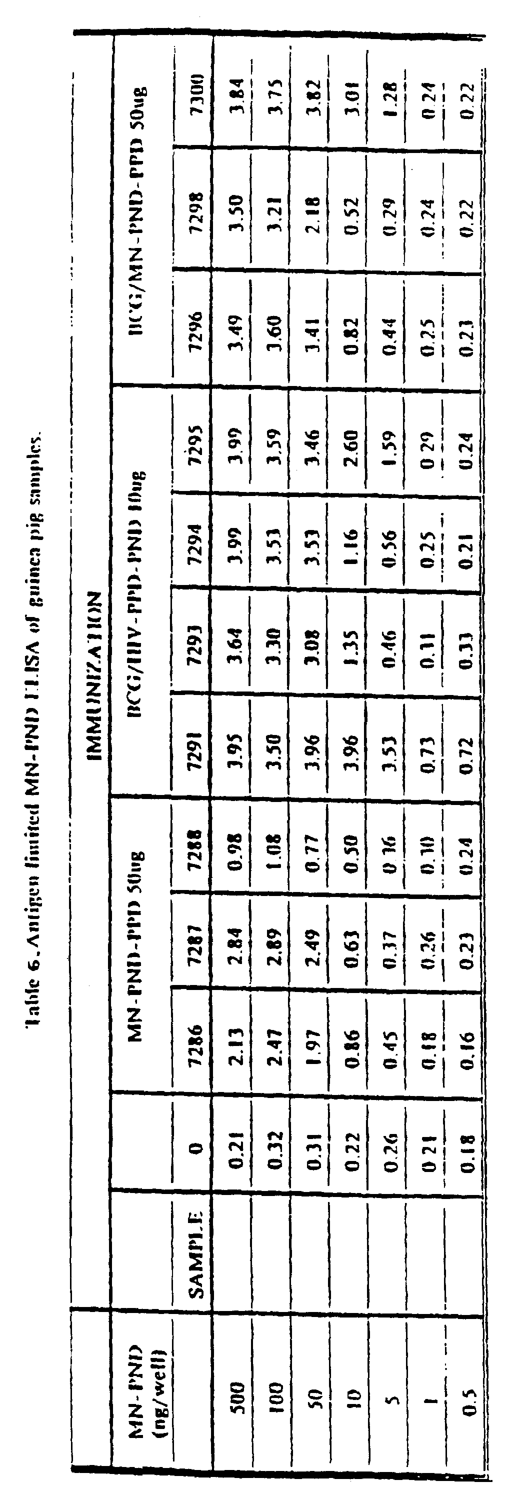

### EXAMPLE VI-Immunization of Humans with Conjugates of Peptides Coupled to Carrier PPD.

Based on the immunogenicity of the PPD-MN-PND conjugates in guinea pigs illustrated in EXAMPLE V, five PPD-positive humans were immunized with this conjugate. The reactivity of sera from the 5 human volunteers was assayed one month after immunization with PPD-MN-PND. No significant IgG or IgM to MN-PND was detected. The sera was again assayed two months after immunization. Table 7 shows the results of the assay.

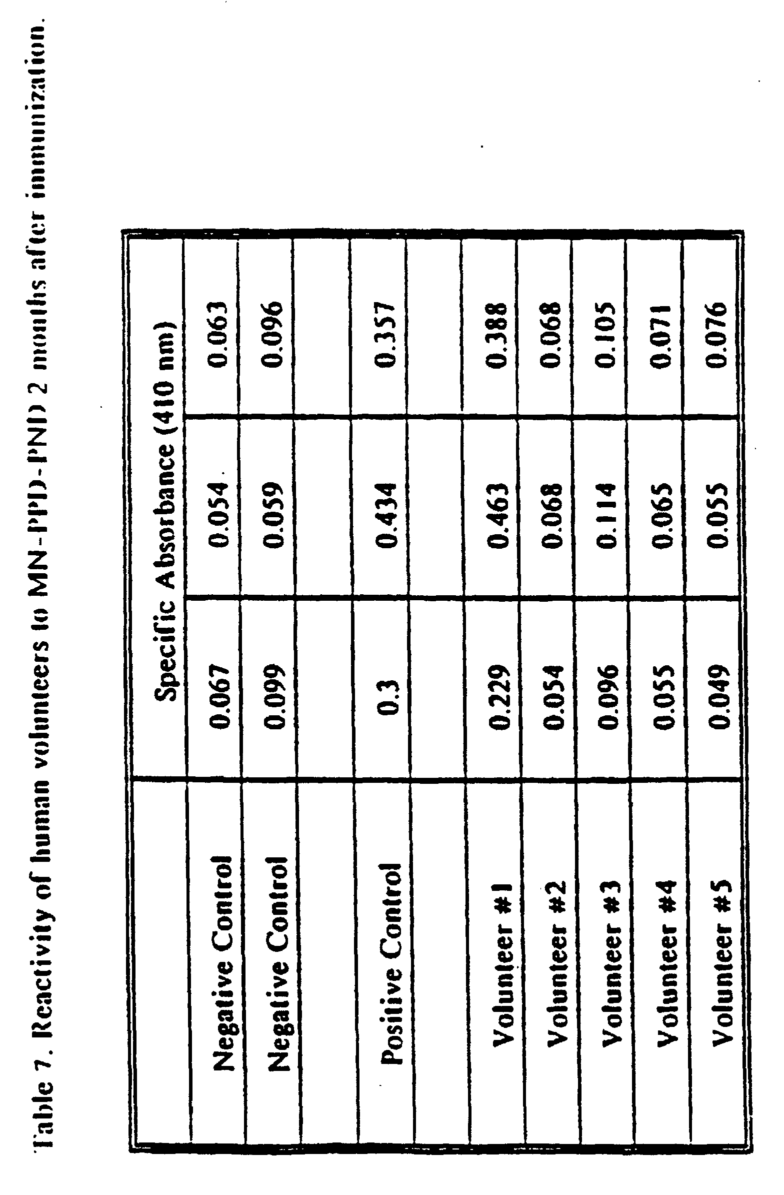

The volunteers were immunized a second time. Serum samples were obtained about 14 days and 28 days after each immunization and prior to immunization. As shown in Table 8 below, after the second immunization, one subject was a very strong responder, one was a "borderline" responder and 2 were non-responders.

**TABLE 8**

| Assay for reactivity to MN-PND was performed in triplicate on the samples obtained 44 days after immunization. | |
|---|---|
| Sample #1- | .370 ± .057 |
| Sample #2- | .063 ± .038 |
| Sample #3- | .105 ± .004 |
| Sample #4- | .063 ± .004 |
| | |
| Positive | .363 + .031 |
| Negative #1 | .084 ± .010 |
| Negative #2 | .061 ± .003 |
| Sample #1 is significantly different from negative controls (p <.001) and sample #3 may be significantly different from negative control (p <.05). | |

The volunteers were immunized a third time. After the third immunization with the PPD-MN-PND conjugate, the serum of one volunteer had a high titer of high affinity/avidity HIV-specific antibodies. Upon exposure to MN-PND, the lymphocytes responded in vitro by proliferation and secretion of interleukin-2. This shows that an entire immune response was induced. B-cell humoral immunity response was induced, as evidenced by the production of antibodies. T-cell immunity response was also induced, as shown by the proliferation of T-cells upon exposure to the conjugate, and as shown by the secretion of interleukin-2, which is a mediator of the immune response. The other three serum samples showed no significant immunological response. The fact that high affinity/avidity HIV-specific antibodies are found more frequently in HIV infected asymptomatic individuals indicates that such antibodies are protective.

The following Examples VII and VIII are not encompassed by the present claims and are maintained for illustrative purposes :

### EXAMPLE VII-Synthesis of Peptides and Immunization of Mice with Conjugates of Synthesized Peptides Coupled to Carrier KLH.

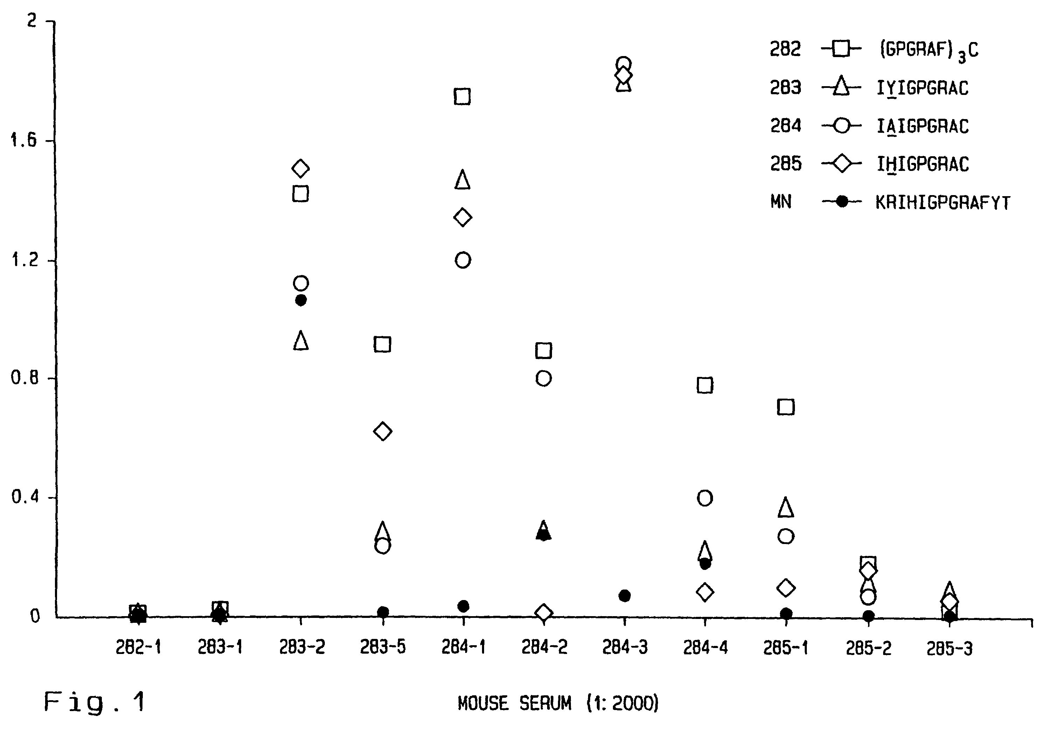

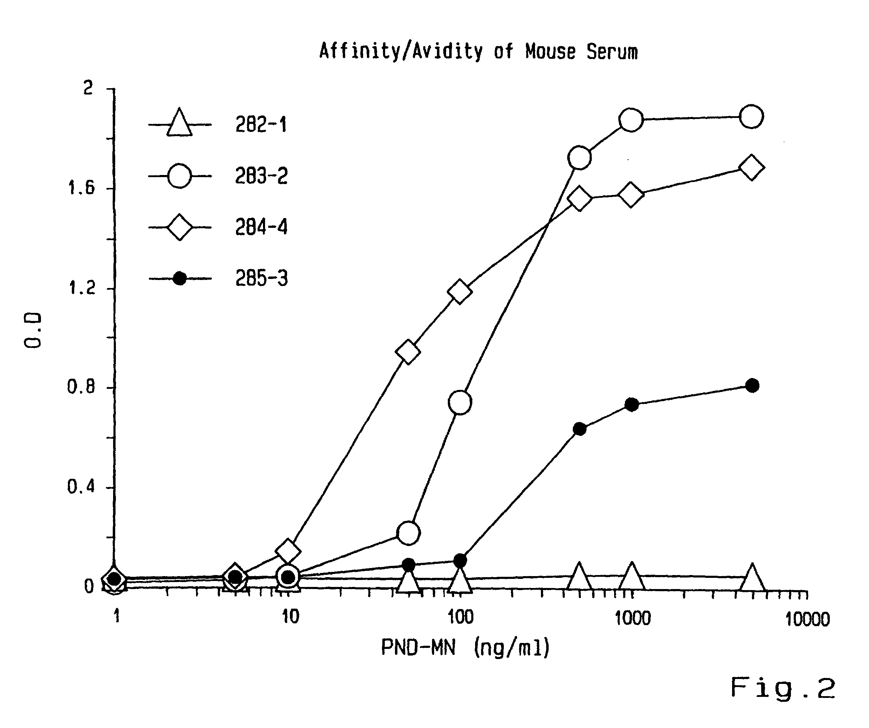

In addition to MN-PND, four other peptides were synthesized. These peptides are 282 (GPGRAFGPGRAFGPGRAFC), 283 (IYIGPGRAC), 284 (IAIGPGRAC) and 285 (IHIGPGRAC). These peptides were coupled to KLH as described above. Five mice were immunized with each peptide conjugate and boosted 2 months later. Serum was obtained. Figure 1 shows the reactivity to each of the peptides. Figure 2 shows the affinity/avidity of the mice with the highest titers of antibody. There was variable immunogenicity of the peptides. 284 was the most highly immunogenic, and 285 was not immunogenic at all. Some mice immunized with 283 and 284 had very low reactivity to MN-PND. This was not just an ELISA artifact as evidenced by the fact that when peptide 282 was incubated with serum from mouse 283-5 prior to performing the ELISA, peptide 282 bound to (competed with) the antibodies in the mouse serum, whereas the MN peptide did not compete with the mouse serum antibodies when incubated therewith. In contrast, peptides 282 and MN both were able to compete with (bind to) the antibodies in the serum of mouse 284-4.

### EXAMPLE VIII-Immunoreactivity of Humans to Synthesized Peptides.

Based on the observation of the differential immunogenicity of the PND peptides, sera from 20 HIV-positive individuals was assessed for reactivity with the five indicated peptides. (See Table 9). Eight samples reacted with at least 4 out of 5 peptides, one sample with 3 out of 5, one sample with 2 out of 5 peptides, and six samples with only 1 out of 5 peptides. One sample was negative, and three samples had high backgrounds. Of eight individuals who had high affinity antibodies to MN-PND, two individuals had high affinity antibodies to 282. In addition, of the eight individuals who had high affinity antibodies to MN-PND, 5 recognized 5 out of 5 peptides, one recognized 4 out of 5 peptides, 1 recognized 3 out of 5 peptides and 1 recognized 1 out of 5 peptides. This indicates that within the group of individuals with high affinity/avidity antibodies to MN-PND, there exist subgroups, some of which may be more protective against HIV. In addition this may have clinical prognostic significance which will affect the inclusion of peptides in a multivalent vaccine.

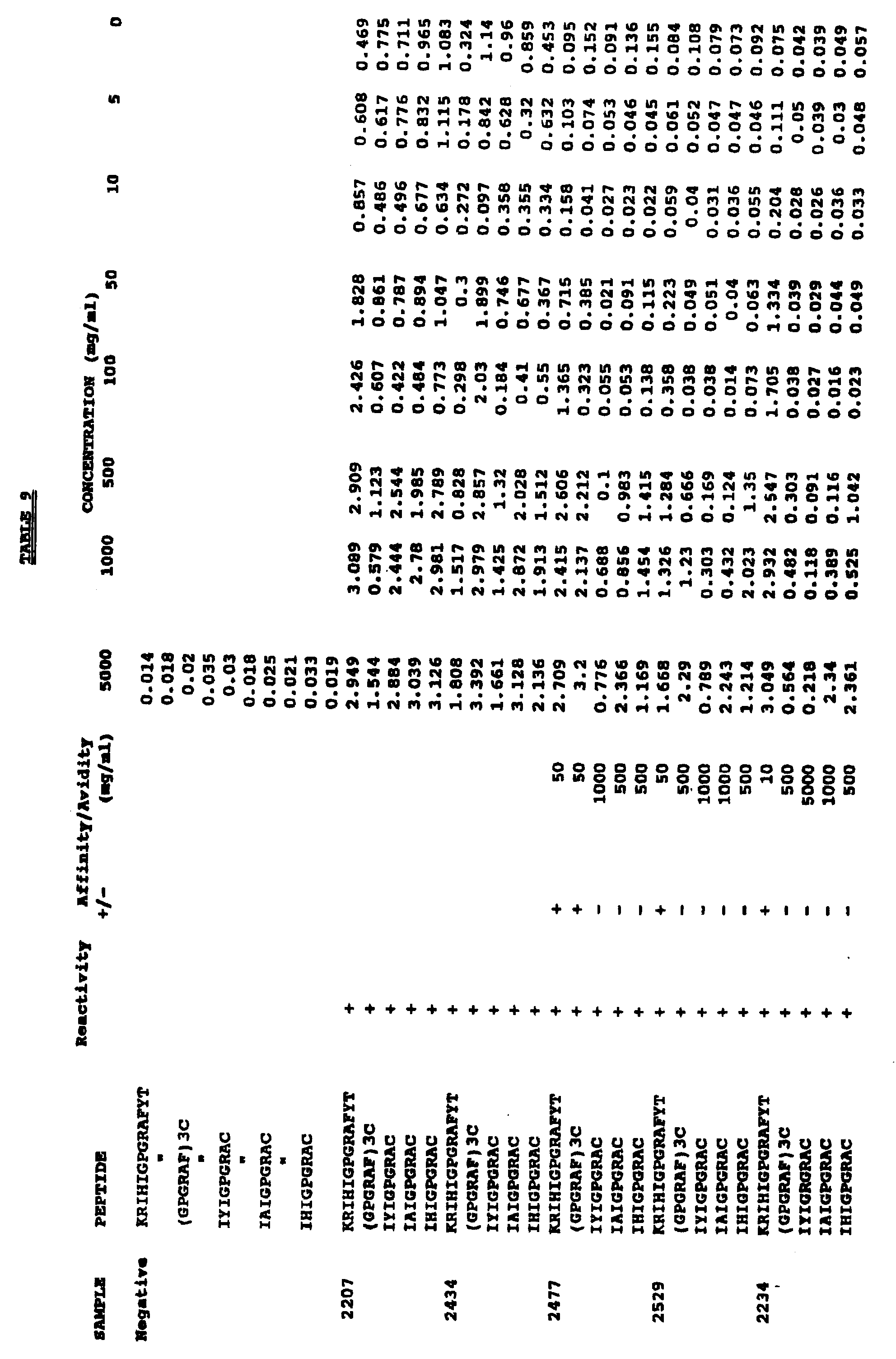

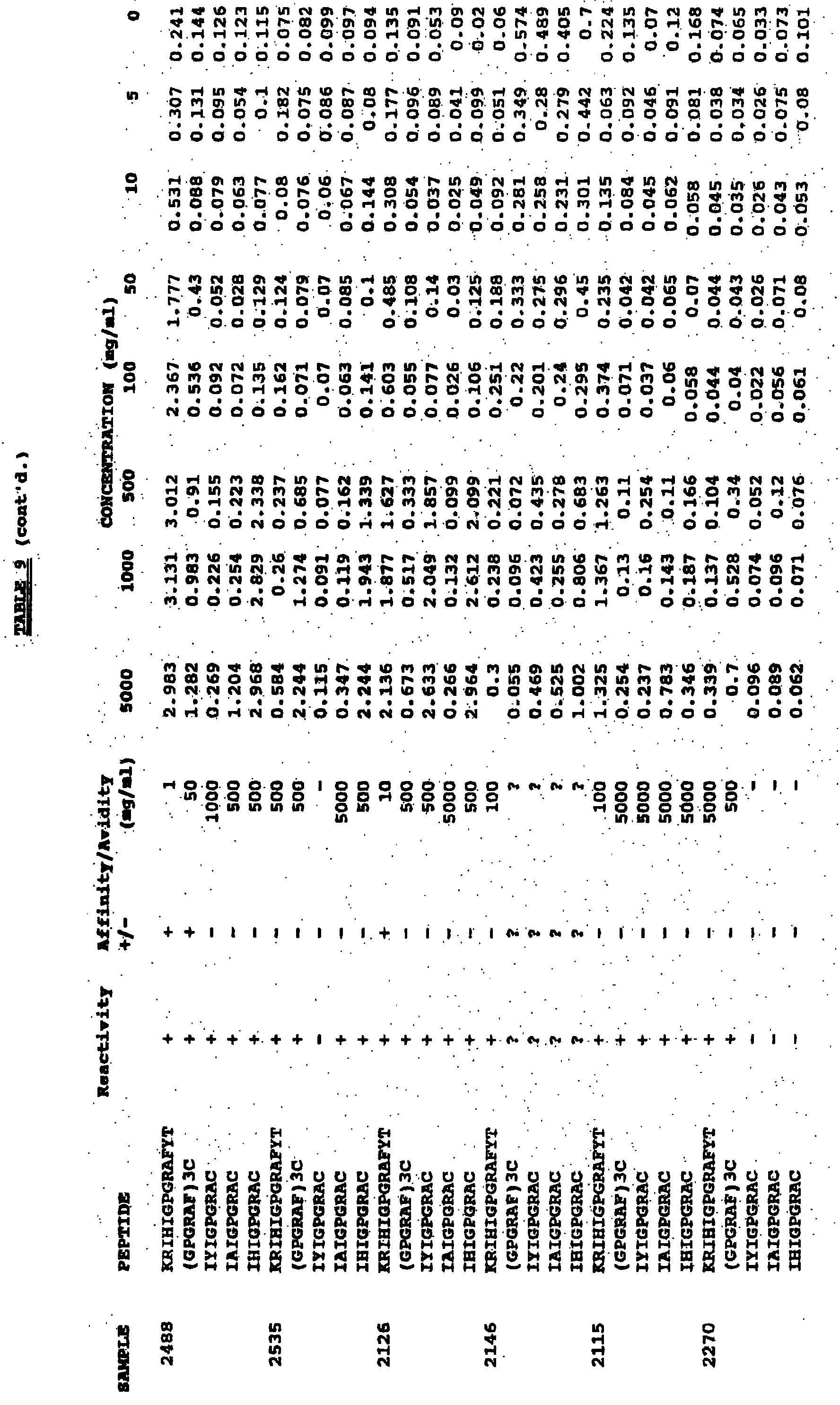

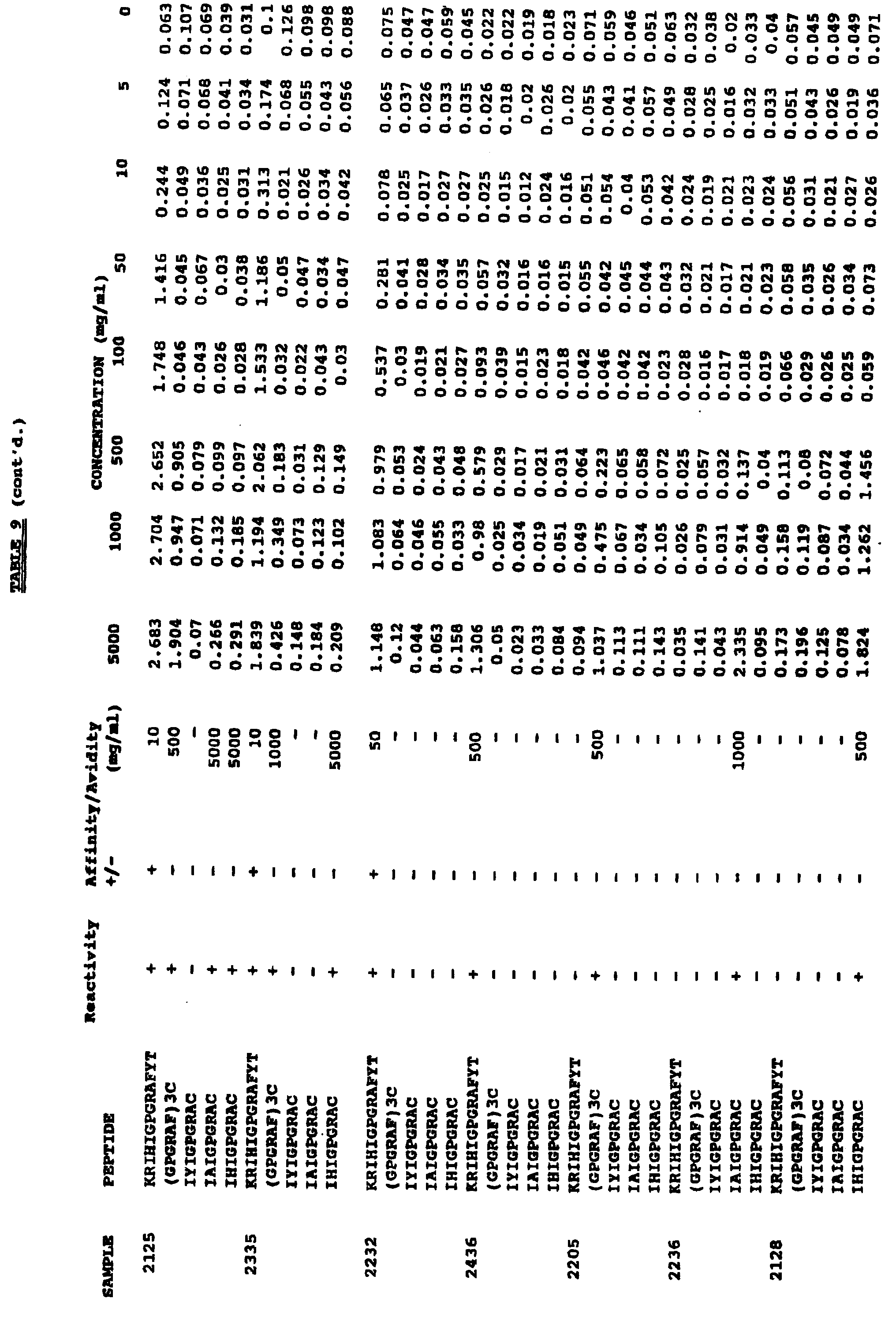

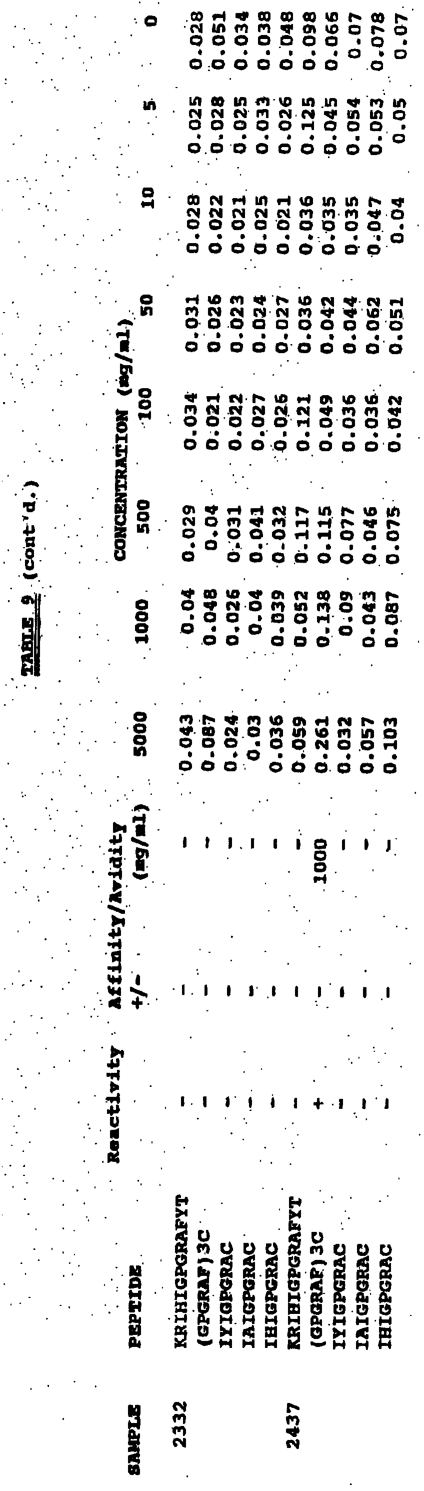

As shown in Table 10, Sample #2234 bound to MN-PND when incubated with MN-PND prior to ELISA, but not with peptide 282. Peptide 282 comprises (GPGRAF)₃C, and the MN peptide contains GPGRAF enclosed by two flanking sequences (KRIHIGPGRAFYT). This implies that the bulk of reactivity to MN-PND is to the flanking sequences. The inability of Sample #2232 to bind to peptide 282 implies that there is only reactivity to flanking sequences and not to GPGRAF. Sample #2434 is difficult to interpret because of high background. Sample #2270 has strong reactivity with peptide 282 in a conjugate, but no reactivity with soluble peptide 282. This suggests the possibility that adhered peptide 282 may express an epitope recognized by the sera that is not present in soluble peptide 282. Another possibility is.that there is a discordance between affinity measured by the antigen-limited ELISA and that measured by competition.

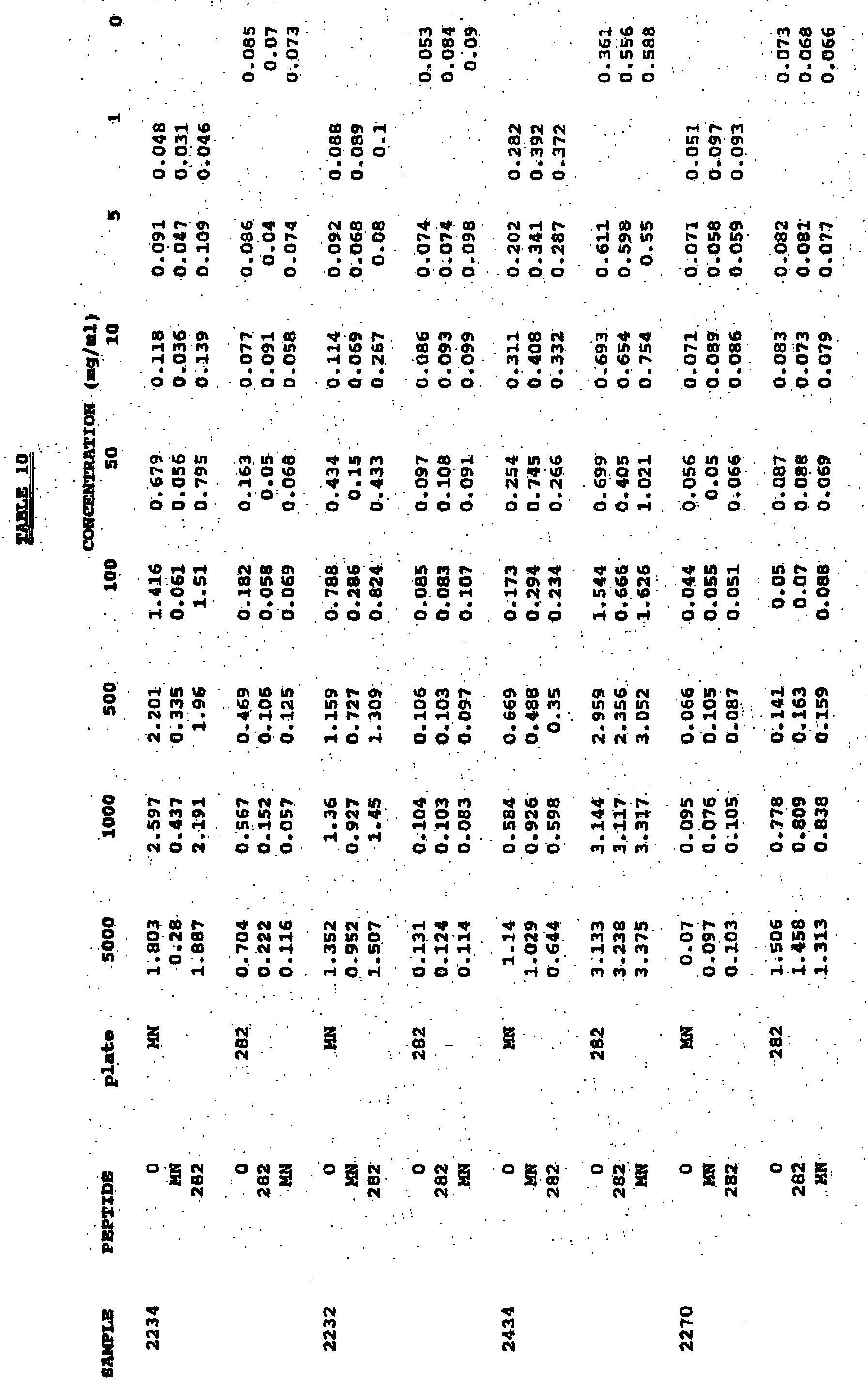

## Claims

1. A vaccine for use in the treatment of HIV-infected and uninfected individuals and/or for the transmission prevention of HIV wherein said vaccine comprises at least the following peptide being coupled to a carrier:
(a) KRIHIGPGRAFYT.

2. The vaccine of claim 1, wherein said vaccine comprises a cocktail of several different peptides being coupled to a carrier wherein the peptides of the cocktail are selected from the group consisting of:
(a) KRIHIGPRAFYT
(b) RHIHIGPGRAFYT
(c) RRIHIGPGRAFYT
(d) TRIHIGPGRAFYT
(e) CTRPNYNKRKRIHIGPGRAFYTTKNIIGTIRQAHC
(f) GPGRAFGPGRAFGPGRAFC
(g) IYIGPGRAC
(h) IAIGPGRAC
(i) IHIGPGRAC
(j) RSIHIGPGRAFYA
(k) KSITKGPGRVIYA
(I) KGIAIGPGRTLYA; and
(m) SRVTLGPGRVWYT.

3. The vaccine of claim 1, wherein the vaccine comprises a cocktail of five or more different peptides coupled to carriers wherein the peptides of the cocktail are selected from the group consisting of:
(a) RHIHIGPGRAFYT
(b) RRIHIGPGRAFYT
(c) TRIHIGPGRAFYT
(d) CTRPNYNKRKRIHIGPGRAFYTTKNIIGTIRQAHC
(e) GPGRAFGPGRAFGPGRAFC
(f) IYIGPGRAC
(g) IAIGPGRAC
(h) IHIGPGRAC
(i) RSIHIGPGRAFYA
(j) KSITKGPGRVIYA
(k) KGIAIGPGRTLYA; and
(I) SRVTLGPGRVWYT.

4. The vaccine of claim 2, wherein the peptides of the cocktail are selected from the group consisting of:
(a) GPGRAFGPGRAFGPGRAFC
(b) IYIGPGRAC
(c) IAIGPGRAC
(d) IHIGPGRAC
(e) RSIHIGPGRAFYA
(f) KSITKGPGRVIYA
(g) KGIAIGPGRTLYA; and
(h) SRVTLGPGRVWYT.

5. The vaccine of any one of claims 1 to 4, wherein said carrier is Pseudomonas aeruginosa exotoxin A, KLH, tetanus toxoid, diptheria toxoid, purified protein derivative (PPD) of tuberculin from Mycobacterium tuberculosis, Bacillus Calmette-Guerin (BCG) or hepatitis B core antigen.

6. The vaccine of any one of claims 1 to 5, wherein the peptide is conjugated to the carrier protein by
(a) a disulfide linkage through a C-terminal peptide cysteine linkage,
(b) reaction of peptide and carrier protein in the presence of glutaraldehyde; or
(c) reaction of peptide and carrier protein in the presence of a watersoluble carbodiimide with or without a spacer molecule.

7. The vaccine of claim 6, wherein the spacer is adipic acid dihydrazide or a bifunctional spacer possessing 2 to 24 carbon atoms between terminal reaction groups.

8. The vaccine of any one of claims 1 to 7, wherein said vaccine is combined with an adjuvant.

9. The vaccine of claim 8, wherein the adjuvant is alumina, Ribi, preferably monophosphoryl lipid A, Freund's, ISCOM's, a virosome or microencapsulation.

10. The vaccine of claim 9, wherein microencapsulation is effected by using polylactide-polyglycolide.

11. The vaccine of claim 9, wherein the alumina comprises either AI(OH)₃ or AIPO₄.

12. Use of a peptide or a cocktail of peptides coupled to carriers as defined in any one of claims 1 to 4 for producing a vaccine for use in the treatment of HIV-Infected and uninfected individuals and/or for the transmission prevention of HIV.

13. A vaccine kit comprising
(a) a vaccine of any one of claims 1 to 11; and
(b) a composition for priming the individual in need thereof before administering said vaccine as a combined preparation for sequential use for the prophylaxis, therapy and/or prevention of transmission of HIV.

14. The vaccine kit of claim 13, wherein said individual is (i) PPD-negative and HIV-negative, or (ii) PPD-negative and HIV-positive but not exhibiting AIDS symptoms and has a CD4 cell count [cells/µl] exceeding 200.

15. The vaccine kit of claim 13, wherein said composition comprises BCG.

16. The vaccine kit of any one of claims 13 to 15, wherein said composition is for priming 4 to 6 weeks prior to the vaccination of the individuals with said vaccine.

## Patentansprüche

1. Impfstoff zur Verwendung in der Behandlung von HIV-infizierten und nicht infizierten Individuen und/oder zur Vermeidung der Übertragung von HIV, wobei der Impfstoff mindestens das folgende an einen Träger gebundene Peptid umfasst:
(a) KRIHIGPGRAFYT.

2. Impfstoff nach Anspruch 1, wobei der Impfstoff ein Gemisch mehrerer verschiedener Peptide umfasst, die an einem Träger gebunden sind, wobei die Peptide des Gemisches ausgewählt sind aus der Gruppe bestehend aus:
(a) KRIHIGPRAFYT
(b) RHIHIGPGRAFYT
(c) RRIHIGPGRAFYT
(d) TRIHIGPGRAFYT
(e) CTRPNYNKRKRIHIGPGRAFYTTKNIIGTIRQAHC
(f) GPGRAFGPGRAFGPGRAFC
(g) IYIGPGRAC
(h) IAIGPGRAC
(i) IHIGPGRAC
(j) RSIHIGPGRAFYA
(k) KSITKGPGRVIYA
(l) KGIAIGPGRTLYA; und
(m) SRVTLGPGRVWYT.

3. Impfstoff nach Anspruch 1, wobei der Impfstoff ein Gemisch von 5 oder mehr verschiedenen Peptiden umfasst, die an Träger gebunden sind, wobei die Peptide des Gemisches ausgewählt sind aus der Gruppe bestehend aus:
(a) RHIHIGPGRAFYT
(b) RRIHIGPGRAFYT
(c) TRIHIGPGRAFYT
(d) CTRPNYNKRKRIHIGPGRAFYTTKNIIGTIRQAHC
(e) GPGRAFGPGRAFGPGRAFC
(f) IYIGPGRAC
(g) IAIGPGRAC
(h) IHIGPGRAC
(i) RSIHIGPGRAFYA
(j) KSITKGPGRVIYA
(k) KGIAIGPGRTLYA; und
(l) SRVTLGPGRVWYT.

4. Impfstoff nach Anspruch 2, wobei die Peptide des Gemisches ausgewählt sind aus der Gruppe bestehend aus:
(a) GPGRAFGPGRAFGPGRAFC
(b) IYIGPGRAC
(c) IAIGPGRAC
(d) IHIGPGRAC
(e) RSIHIGPGRAFYA
(f) KSITKGPGRVIYA
(g) KGIAIGPGRTLYA; und
(h) SRVTLGPGRVWYT.

5. Impfstoff nach einem der Ansprüche 1 bis 4, wobei der Träger Pseudomonas aeruginosa-Exotoxin A, KLH, Tetanus-Toxoid, Diphteria-Toxoid, gereinigtes Protein-Derivat (PPD) von Tuberculin von Mycobacterium tuberculosis, Bacillus Calmette-Guerin (BCG) oder Hepatitis -B-Kern-Antigen ist.

6. Impfstoff nach einem der Ansprüche 1 bis 5, wobei das Peptid an das Trägerprotein konjugiert ist über
(a) eine Disulfidbindung über eine C-terminale Peptid-Cystein-Bindung,
(b) Reaktion des Peptids und des Trägerproteins in Anwesenheit von Glutaraldehyd; oder
(c) Reaktion des Peptids und des Trägerproteins in Anwesenheit eines wasserlöslichen Carbodiimids mit oder ohne Spacer-Molekül.

7. Impfstoff nach Anspruch 6, wobei der Spacer ein Adipinsäuredihydrazid oder ein bifunktioneller Spacer ist, der 2 bis 24 Kohlenstoffatome zwischen den endständigen Reaktionsgruppen besitzt.

8. Impstoff nach einem der Ansprüche 1 bis 7, wobei der Impstoff mit einem Adjuvans kombiniert ist.

9. Impfstoff nach Anpruch 8, wobei das Adjuvans Aluminiumoxid, Ribi, vorzugsweise Monophosphoryl-Lipid A, Freundsches, ISCOM's, ein Virosom oder eine Mikroverkapselung ist.

10. Impfstoff nach Anspruch 9, wobei die Mikroverkapselung durch Verwendung von Polylactid-Polyglycolid bewirkt wird.

11. Impfstoff nach Anspruch 9, wobei das Aluminiumoxid entweder Al(OH)₃ oder AlPO₄ umfasst.

12. Verwendung eines Peptids oder Gemisches von Peptiden, die an Träger gebunden sind wie in einem der Ansprüche 1 bis 4 definiert, zur Herstellung eines Impfstoffs zur Verwendung in der Behandlung von HIV-infizierten und nicht infizierten Individuen und/oder zur Vermeidung der Übertragung von HIV.

13. Impfstoff-Kit umfassend:
(a) einen Impfstoff nach einem derAnsprüche 1 bis 11; und
(b) eine Zusammensetzung zum Primen des Individuums, das es nötig hat, vor dem Verabreichen des Impfstoffs als kombinierte Zubereitung zur sequentiellen Verwendung für die Prophylaxe, Therapie und/oder Vermeidung der Übertragung von HIV.

14. Impfstoff-Kit nach Anspruch 13, wobei das Individuum (i) PPD-negativ und HIV-negativ oder (ii) PPD-negativ und HIV-positiv ist, jedoch keine AIDS-Symptome zeigt und eine CD4-Zellzahl [Zellen/µl] hat, die 200 überschreitet.

15. Impfstoff-Kit nach Anspruch 13, wobei die Zusammensetzung BCG umfasst.

16. Impfstoff-Kit nach einem der Ansprüche 13 bis 15, wobei die Zusammensetzung zum Primen 4 bis 6 Wochen vor der Impfung der Individuen mit dem Impfstoff dient.

## Revendications

1. Vaccin pour une utilisation dans le traitement d'individus infectés et non-infectés par le VIH et/ou pour la prévention de la transmission du VIH, dans lequel ledit vaccin comprend au moins le peptide suivant couplé à un porteur :
(a) KRIHIGPGRAFYT.

2. Vaccin selon la revendication 1, dans lequel ledit vaccin comprend un cocktail de plusieurs peptides différents couplés à un porteur, les peptides du cocktail étant choisis dans le groupe constitué de :
(a) KRIHIGPRAFYT,
(b) RHIHIGPGRAFYT
(c) RRIHIGPGRAFYT
(d) TRIHIGPGRAFYT
(e) CTRPNYNKRKRIHIGPGRAFYTTKNIIGTIRQAHC
(f) GPGRAFGPGRAFGPGRAFC
(g) IYIGPGRAC
(h) IAIGPGRAC
(i) IHIGPGRAC
(j) RSIHIGPGRAFYA
(k) KSITKGPGRVIYA
(1) KGIAIGPGRTLYA ; et
(m) SRVTLGPGRVWYT.

3. Vaccin selon la revendication 1, dans lequel ledit vaccin comprend un cocktail de cinq ou plus peptides différents couplés à des porteurs, les peptides du cocktail étant choisis dans le groupe constitué de :
(a) RHIHIGPGRAFYT
(b) RRIHIGPGRAFYT
(c) TRIHIGPGRAFYT
(d) CTRPNYNKRKRIHIGPGRAFYTTKNIIGTIRQAHC
(e) GPGRAFGPGRAFGPGRAFC
(f) IYIGPGRAC
(g) IAIGPGRAC
(h) IHIGPGRAC
(i) RSIHIGPGRAFYA
(j) KSITKGPGRVIYA
(k) KGIAIGPGRTLYA ; et
(1) SRVTLGPGRVWYT.

4. Vaccin selon la revendication 2, dans lequel les peptides du cocktail sont choisis dans le groupe constitué de :
(a) GPGRAFGPGRAFGPGRAFC
(b) IYIGPGRAC
(c) IAIGPGRAC
(d) IHIGPGRAC
(e) RSIHIGPGRAFYA
(f) KSITKGPGRVIYA
(g) KGIAIGPGRTLYA ; et
(h) SRVTLGPGRVWYT.

5. Vaccin selon l'une quelconque des revendications 1 à 4, dans lequel ledit porteur est l'exotoxine A de Pseudomonas aeruginosa, KLH, un toxoïde de tétanos, un toxoïde diphtérique, un dérivé protéique purifié (DPP) de la tuberculine de Mycobactérium tuberculosis, du bacille Calmette-Guérin (BCG) ou de l'antigène core de l'hépatite B.

6. Vaccin selon l'une quelconque des revendications 1 à 5, dans lequel le peptide est conjugué à la protéine porteur par :
(a) un pontage disulfure par liaison avec une cystéine peptidique C-terminale,
(b) réaction du peptide et de la protéine porteur en présence de glutaraldéhyde ; ou
(c) réaction du peptide et de la protéine porteur en présence de carbodiimide soluble dans l'eau, avec ou sans molécule espaceur.

7. Vaccin selon la revendication 6, dans lequel l'espaceur est le dihydrazide de l'acide adipique ou un espaceur bi-fonctionnel ayant de 2 à 24 atomes de carbone entre des groupes réactifs terminaux.

8. Vaccin selon l'une quelconque des revendications 1 à 7, dans lequel ledit vaccin est combiné à un adjuvant.

9. Vaccin selon la revendication 8, dans lequel l'adjuvant est l'alumine, Ribi, de préférence lipide A monophosphorylé, l'adjuvant de Freund, d'ISCOM, un virosome ou la microencapsulation.

10. Vaccin selon la revendication 9, dans lequel la microencapsulation est réalisée en utilisant un polylactide-polyglycolide.

11. Vaccin selon la revendication 9, dans lequel l'alumine comprend Al(OH)₃ ou AlPO₄.

12. Utilisation d'un peptide ou d'un cocktail de peptides couplés à des porteurs tels que définis dans l'une quelconque des revendications 1 à 4 pour la production d'un vaccin pour une utilisation dans le traitement d'individus infectés et non-infectés par le VIH et/ou pour la prévention de la transmission du VIH.

13. Un kit de vaccin comprenant :
(a) un vaccin selon l'une quelconque des revendications 1 à 11 ; et
(b) une composition pour stimuler l'individu en besoin, avant l'administration dudit vaccin, en tant que préparation combinée pour une utilisation séquentielle pour la prophylaxie, la thérapie et/ou la prévention de la transmission du VIH.

14. Kit de vaccin selon la revendication 13, dans lequel ledit individu est (i) PDD-négatif et VIH-négatif, ou (ii) PDD-négatif et VIH-positif mais ne présentant pas les symptômes du SIDA et possède un taux de cellules CD4 [cellules/µl] supérieur à 200.

15. Kit de vaccin selon la revendication 13, dans lequel ladite composition comprend du BCG.

16. Kit de vaccin selon l'une quelconque des revendications 13 à 15, dans lequel ladite composition est destinée à stimuler 4 à 6 semaines avant la vaccination de l'individu avec ledit vaccin.


### SEQUENCE LISTING

(1) GENERAL INFORMATION
   (i) APPLICANT:
      Arye Rubinstein
      Barry Bloom
      Yair Devash
      Stanley Cryz
   (ii) TITLE OF INVENTION: Therapeutic and Preventive Vaccines for HIV
   (iii) NUMBER OF SEQUENCES: 9
   (iv) CORRESPONDENCE ADDRESS:
      (A) ADDRESSEE: Amster, Rothstein & Ebenstein
      (B) STREET: 90 Park Avenue
      (C) CITY: New York
      (D) STATE: New York
      (E) COUNTRY: U.S.A.
      (F) ZIP: 10016
   (v) COMPUTER READABLE FORM:
      (A) MEDIUM TYPE: 3.5 inch 1.44 Mb storage diskette
      (B) COMPUTER: IBM PC Compatible
      (C) OPERATING SYSTEM: MS-DOS
      (D) SOFTWARE: Word Processor (ASCII)
   (vi) CURRENT APPLICATION DATA:
      (A) APPLICATION NUMBER: Not Yet Assigned
      (B) FILING DATE: Not Yet Assigned
      (C) CLASSIFICATION: Not Yet Assigned
   (vii) PRIOR APPLICATION DATA: Continuation-In-Part of Application Serial No. 07/681,624 filed April 2, 1991, entitled THERAPEUTIC AND PREVENTIVE VACCINES FOR HIV
   (viii) ATTORNEY/AGENT INFORMATION:
      (A) NAME: Pasqualini, Patricia A.
      (B) REGISTRATION NUMBER: 34,894
      (C) REFERENCE/DOCKET NUMBER: 96700/188
   (ix) TELECOMMUNICATION INFORMATION:
      (A) TELEPHONE: (212) 697-5995
      (B) TELEFAX: (212) 286-0854 or 286-0082
      (C) TELEX: TWX 710-581-4766
(2) INFORMATION FOR SEQ. ID NO: 1
   (i) SEQUENCE CHARACTERISTICS:
      (A) LENGTH: 19 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 1
   Gly Pro Gly Arg Ala Phe Gly Pro Gly Arg Ala Phe Gly Pro Gly Arg
    1               5                   10                  15
   Ala Phe Cys
(3) INFORMATION FOR SEQ. ID NO: 2
   (i) SEQUENCE CHARACTERISTICS:
      (A) LENGTH: 9 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 2
   Ile Tyr Ile Gly Pro Gly Arg Ala Cys
    1               5
(4) INFORMATION FOR SEQ. ID NO: 3
   (i) SEQUENCE CHARACTERISTICS:
      (A) LENGTH: 9 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 3
   Ile Ala Ile Gly Pro Gly Arg Ala Cys
    1               5
(5) INFORMATION FOR SEQ. ID NO: 4
   (i) SEQUENCE CHARACTERISTICS:
      (A) LENGTH: 9 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 4
   Ile His Ile Gly Pro Gly Arg Ala Cys
    1               5
(6) INFORMATION FOR SEQ. ID NO: 5
   (i) SEQUENCE CHARACTERISTICS:
      (A) LENGTH: 13 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 5
   Lys Arg Ile His Ile Gly Pro Gly Arg Ala Phe Tyr Thr
    1               5                  10
(7) INFORMATION FOR SEQ. ID NO: 6
   (i) SEQUENCE CHARACTERISTICS:
      (A) LENGTH: 13 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE : internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 6
   Arg Ser Ile His Ile Gly Pro Gly Arg Ala Phe Tyr Ala
    1               5                   10
(8) INFORMATION FOR SEQ. ID NO: 7
   (i) SEQUENCE CHARACTERISTICS:
      (A) LENGTH: 13 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 7
   Lys Ser Ile Thr Lys Gly Pro Gly Arg Val Ile Tyr Ala
    1               5                  10
(9) INFORMATION FOR SEQ. ID NO: 8
   (i) SEQUENCE CHARACTERISTICS:
      (A) LENGTH : 13 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 8
   Lys Gly Ile Ala Ile Gly Pro Gly Arg Thr Leu Tyr Ala
    1               5                  10
(10) INFORMATION FOR SEQ. ID NO: 9
   (i) SEQUENCE CHARACTERISTICS:
      (A) LENGTH: 13 amino acids
      (B) TYPE: amino acid
      (C) STRANDEDNESS: single
      (D) TOPOLOGY: linear and circular
   (ii) MOLECULE TYPE:
      (A) DESCRIPTION: peptide
   (iii) HYPOTHETICAL: No
   (iv) ANTI-SENSE: No
   (v) FRAGMENT TYPE: internal fragment
   (vi) ORIGINAL SOURCE:
      (A) ORGANISM: HIV
      (B) STRAIN: MN family
      (C) INDIVIDUAL ISOLATE: not applicable
      (D) DEVELOPMENTAL STAGE: not applicable
      (E) HAPLOTYPE: not applicable
      (F) TISSUE TYPE: not applicable
      (G) CELL TYPE: not applicable
      (H) CELL LINE: not applicable
      (I) ORGANELLE: not applicable
   (vii) IMMEDIATE SOURCE: not applicable
   (viii) POSITION IN GENOME:
      (A) CHROMOSOME SEGMENT: part of V3 loop fragment of envelope
   (ix) FEATURE:
      (A) NAME/KEY: peptide
      (B) LOCATION: PND of HIV
      (C) IDENTIFICATION METHOD: not applicable
      (D) OTHER INFORMATION: not applicable
   (x) PUBLICATION INFORMATION: none
      (A) AUTHORS: none
      (B) TITLE: none
      (C) JOURNAL: none
      (D) VOLUME: none
      (F) PAGES: none
      (G) DATE: none
      (H) DOCUMENT NUMBER: none
      (I) FILING DATE: none
      (J) PUBLICATION DATE: none
      (K) RELEVANT RESIDUES: none
   (xi) SEQUENCE DESCRIPTION: SEQUENCE ID NO: 9
   Ser Arg Val Thr Leu Gly Pro Gly Arg Val Trp Tyr Thr
    1               5                  10